# EUROPÄISCHE PATENTSCHRIFT

(11) **EP 0 997 724 B1**
(45) Veröffentlichungstag und Bekanntmachung des Hinweises auf die Patenterteilung: **09.01.2002**
(21) Anmeldenummer: 99119488.7
(22) Anmeldetag: 30.09.1999
(51) Int. Cl.: G01M 11/00, A61B 1/07

(54) **Vorrichtung zur Prüfung und Zustandsbeurteilung von Endoskopen**
Device for testing and for evaluating the condition of endoscopes
Dispositif pour essayer et pour évaluer l'état des endoscopes

(30) Priorität: 29.10.1998 DE 29819269 U
(43) Veröffentlichungstag der Anmeldung: 03.05.2000
(73) Patentinhaber: Hilger, Claus, 36093 Künzell (DE)
(72) Erfinder: Hilger, Claus, 36093 Künzell (DE)
(74) Vertreter: RACKETTE Partnerschaft Patentanwälte

(56) Entgegenhaltungen:
- US-A- 5 820 547

## Beschreibung

Die Erfindung betrifft eine Vorrichtung zur Prüfung und Zustandsbeurteilung von Endoskopen.

Die Endoskopie hat als Untersuchungsmethode von Körperöffnungen oder Körperhöhlen durch röhren- oder schlauchförmige optische Systeme in der Medizin einen bedeutenden Stellenwert erlangt. Mit ihrer Hilfe ist es dem behandelnden Arzt möglich, Organe, Schleimhäute, verdächtiges Gewebe oder dergleichen im Körper des Patienten ohne beziehungsweise durch sehr kleine (minimal-invasive) operative Eingriffe zu betrachten. Die dabei gewonnenen Bilder stellen oftmals die Grundlage für eine Behandlungsmethode dar, die einen mitunter risikoreicheren chirurgischen Eingriff überflüssig macht. Ist eine Operation unumgänglich, dient die sogenannte intraoperative Endoskopie dem Chirurgen während des Eingriffs als unverzichtbares Hilfsmittel.

Die zur Endoskopie verwendeten Endoskope sind aufgrund von Aufbereitungsverfahren wie Sterilisation oder dergleichen, die vor ihrem Einsatz durchzuführen sind, hohen Belastungen ausgesetzt. Erfahrungsgemäß verschlechtert sich ihre Bildqualität über eine längere Betriebsdauer hinweg und die von ihnen erzeugten Bilder werden milchig oder trübe.

Ursächlich für das Auftreten von Bildeintrübungen starrer Endoskope mit einem Stablinsen beinhaltenden starren Hüllrohr sind beispielsweise Linsenbrüche, die infolge mechanischer Beschädigungen des Hüllrohres entstehen. Die bis zu 50 mm langen Stablinsen sind oftmals spröde und zerbrechen demzufolge leicht, so daß bereits leichte Verformungen des Hüllrohres die Abbildungsqualität eines Endoskopes beeinträchtigen können. Oftmals werden sogar Linsenbrüche beobachtet, ohne daß mechanische Beschädigungen wie Verbiegungen oder Dellen am Hüllrohr feststellbar sind.

Neben Linsenbrüchen bewirken Eintrübungen des optischen Systems eine Verschlechterung der Bildqualität. Verunreinigungen und Niederschläge, die durch Undichtigkeiten in das Endoskop eingedrungen sind, schlagen sich auf den optischen Komponenten nieder und verändern dadurch optische Parameter wie beispielsweise Transmission, Kontrast und Bildschärfe. Starke Temperaturschwankungen, denen Endoskope beispielsweise bei einer Sterilisation oftmals ausgesetzt sind, verursachen eine Bewegungen der Stablinsen und Abstandshülsen, die zwischen den Stablinsen angeordnet sind, bezüglich des Hüllrohres. Auf diese Weise kommt es zu Verschleißerscheinungen in Form von Materialabrieb. Ferner neigen Kitte, die zur Verbindung von Stablinsen verwendet werden, insbesondere bei hohen Temperaturen zum Eintrüben und beeinflussen somit ebenfalls die Bildqualität.

In der Praxis ist sinnvoll, zwischen Verschlechterungen der Bildqualität zu unterscheiden, die aufgrund mechanischer Beschädigungen entstehen, und solchen, die bei sachgerechter Nutzung des Endoskopes als natürlicher Verschleiß auftreten. Beispielsweise wäre im Hinblick auf die Inanspruchnahme von Leistungen, die aus einem Garantievertrag heraus entstehen, ein Prüfverfahren wünschenswert, das eine Differenzierung der genannten Schadensursachen erlaubt. Ein weiterer sinnvoller Nutzen einer solchen Vorrichtung entsteht bei häufigen Optikausfällen infolge einer mechanischen Beschädigung, so daß Untersuchungen nach dem Verursacher erforderlich sind. Durch das Prüfverfahren kann der Personenkreis, der den Schaden verursacht haben könnte, eingegrenzt werden. Mit Hilfe eines derartigen Prüfverfahrens könnte weiterhin das Sterilisationsverfahren des Endoskopes dahingehend optimiert werden, daß die trotz sachgerechter Benutzung aufgetretenen Verschleißerscheinungen minimiert sind.

Vorbekannte Maßnahmen der präoperativen optischen Qualitäts- und Funktionsprüfung umfassen die visuelle Überprüfung der Optiken gegen Ende eines Aufbereitungsprozesses, wobei der Bildeindruck der Optiken in dem Endoskop durch einfaches Hindurchsehen überprüft und das Hüllrohr anschließend auf mechanische Beschädigungen untersucht wird. Nachteilig bei diesem Verfahren ist jedoch der große Ermessensspielraum des Untersuchenden sowie die Unsicherheiten, die sich im Hinblick auf langsam ablaufende Veränderungen, wie sie bei der Mehrzahl der verschleißbedingten Eintrübungen typisch sind, einstellen. Darüber hinaus können gemäß den vorhergehenden Ausführungen Linsenschäden als Folge mechanischer Beschädigungen auftreten, ohne daß letztere am Hüllrohr feststellbar wären, weil diese im elastischen Bereich der Verformung dieser Komponente abgelaufen sind.

Bei einem weiteren vorbekannten Prüfverfahren wird der Transmissionsgrad des Endoskopes bestimmt. Die zur Durchführung des Verfahrens verwendete Vorrichtung umfaßt ein Leuchtdichtemeßgerät zum Anschluß an das Okular des Endoskopes, das die durch die Optik des Endoskopes hindurchtretende Lichtmenge mißt. Die auf diese Weise gewonnenen Meßwerte werden anschließend in ein Verhältnis zu einem zuvor gemessenen Referenzwert gesetzt und so der Transmissionsgrad der Optiken bestimmt. Zwar weist ein hoher Transmissionsgrad neben anderen Güteparametern auf eine gute Bildqualität hin. Aufgrund bauartbedingter Lichtverluste ist der Transmissionsgrad neuwertiger Endoskope jedoch geringer als 100 Prozent, so daß der Transmissionsgrad neuer Endoskope als Fixpunkt zur Beurteilung der Güte des Endoskopes durch Messen zu ermitteln ist. Weiterhin muß festgelegt werden, ab welchem Transmissionsgrad das Endoskopiegerät als unbrauchbar gilt und für weitere Untersuchungen nicht mehr einsetzbar ist. Die Transmissionsmethode stellt demnach erst nach aufwendigen Vorarbeiten ein Auswahlkriterium für starre Endoskope bereit. Darüber hinaus gestattet die Angabe eines Transmissionsgrades nicht, zwischen Transmissionsverlusten aufgrund von Eintrübungen oder Linsenbrüchen zu unterscheiden.

In der Praxis wird daher als optisches Verfahren zur Prüfung von Endoskopen eine Augenlupe verwendet. Dazu wird das Endoskop gegen eine hell reflektierende Fläche gerichtet und mit der Augenlupe in das Okular des Endoskopes geschaut. Um eine Stablinsenoberfläche des Endoskopes auf diese Weise auf der Netzhaut abzubilden, sind die optischen Achsen des Auges und der Augenlupe bezüglich der Längsachse des Endoskopes von Hand auszurichten. Die Variation des Abstandes zwischen Endoskop und Augenlupe unter Beibehaltung der Achsenausrichtung erlaubt das Betrachten sämtlicher Stablinsen im Endoskopinneren. Die Bildvergrößerung der Augenlupe ermöglicht eine Differenzierung zwischen Verunreinigungen des optischen Systems und Linsenbrüchen. Nachteilig bei dem beschriebenen Verfahren wirkt sich jedoch das aufwendige Ausrichten der besagten optischen Achsen bezüglich der Endoskoplängsachse aus, so daß die Ergebnisse oftmals nicht reproduzierbar und unter großen Ermüdungserscheinungen nur von erfahrenem Personal durchführbar sind.

In der optischen Industrie werden zur Gütebestimmung von Endoskopen optische Bänke mit handelsüblichen optischen Bauteilen verwendet, mit deren Hilfe die Endoskope exakt bezüglich der optischen Achse einer Auswerteeinheit wie beispielsweise einer Kamera ausgerichtet und untersucht werden. Diese optischen Bauteile und Tische sind jedoch kostspielig und sperrig, so daß sie an häufig wechselnden Einsatzorten von Endoskopen wie beispielsweise in einer Klinik nicht verwendbar sind.

Aufgabe der Erfindung ist es daher, ein Vorrichtung bereitzustellen, die ein einfache, kostengünstige und schnelle Prüfung sowie Zustandsbeurteilung von Endoskopen erlaubt, die von ungeschultem Personal bedienbar ist und reproduzierbare Prüfergebnisse liefert.

Die Aufgabe wird erfindungsgemäß mit einer Vorrichtung zur Prüfung und Zustandsbeurteilung von Endoskopen mit einer zur Aufnahme des zu prüfenden Endoskopes vorgesehen Führungseinheit, einer Linsenanordnung, die als Okular am proximalen Ende der Führungseinheit angeordnet ist, und einem in der Führungseinheit in Richtung der optischen Achse der Linsenordnung verfahrbaren Halteteil zur Aufnahme und Fixierung des Endoskopes gelöst.

Mit Hilfe der erfindungsgemäßen Vorrichtung ist es möglich, Endoskope auf einfache Art und Weise auf ihre Bildqualität hin zu untersuchen, wobei aufgrund der Verfahrbarkeit des Halteteils entlang der optischen Achse ein aufwendiges Ausrichten von Hand entfällt. Die erfindungsgemäße Vorrichtung ist daher leicht handhabbar, so daß auch ungeschultes Personal zur Prüfung von Endoskopen eingesetzt werden kann. Weiterhin sind die Prüfungsergebnisse durch das vereinfachte Ausrichten reproduzierbar und die Herstellungskosten der erfindungsgemäßen Vorrichtung im Vergleich zu den beschriebenen vorbekannten Prüfvorrichtungen mit reproduzierbaren Prüfungsergebnissen gering.

In einem bevorzugten Ausführungsbeispiel ist die Führungseinheit als Führungsrohr ausgebildet, wobei das Halteteil vorteilhafterweise ein kreiszylinderförmiger Gleitkörper ist, dessen Außendurchmesser an den Innendurchmesser des Führungsrohres angepaßt ist und der eine mittige Durchgangsöffnung zum Hindurchführen und Fixieren eines Hüllrohres des zu prüfenden Endoskopes aufweist. Dabei ist der Innendurchmesser der Durchgangsöffnung zweckmäßigerweise an den Außendurchmesser des Hüllrohres angepaßt.

Vorteilhafterweise weist der Gleitkörper eine in seiner Längsrichtung verlaufende Nut auf, die mit ihrer Bodenwandung an einem in der Führungsrohrwandung drehbar gelagerten Schieberad anliegt, so daß der Gleitkörper reibschlüssig mit dem Verschieberad verbunden ist, wobei das Verschieberad die Führungsrohrwandung umfänglich überragt und durch seine von Hand antreibbare Drehung das Verfahren des Gleitkörpers in dem Führungsrohr bewirkt.

Davon abweichend kann die Nut vorteilhafterweise einen Zahnprofilbereich aufweisen und das Verschieberad als ein in den Zahnprofilbereich eingreifendes Zahnrad ausgebildet sein, wobei das Zahnrad über eine Welle drehfest mit einem außerhalb des Führungsrohrs gelagerten Stellrad verbunden ist.

Eine besonders genaue Einstellung der Bildschärfe ist erzeugbar, wenn die Stellradwelle mit einer auf dem Führungsrohr befestigten Antriebseinheit gekoppelt ist, die einen von einem Energiespeicher gespeisten Elektromotor, Getriebemittel und einen aus der Antriebseinheit hervorragenden, federnden Druckkontakt umfaßt, der während eines von außen aufgebrachten Druckes, beispielsweise von Hand, das Einleiten einer von dem Elektromotor erzeugten Drehbewegung auf die Stellradwelle und damit eine Verschiebung des Gleitkörpers in dem Führungsrohr bewirkt.

Zweckmäßigerweise verfügt das Führungselement über zumindest eine in seine Rohrwandung eingeschnittene Ausnehmung, die sich von seinem distalen Ende in Längsrichtung erstreckt. In einer diesbezüglichen Weiterentwicklung erstreckt sich zumindest eine Ausnehmung durchgängig, über das gesamte Führungselement hinweg. In einem davon abweichenden Ausführungsbeispiel der erfindungsgemäßen Vorrichtung erstreckt sich zumindest eine Ausnehmung lediglich bis zur Linsenanordnung.

In einem bevorzugten Ausführungsbeispiel ist das Führungselement und das Halteteil aus einem lichtabschirmenden Material hergestellt, um einen störenden Einfall von Fremdlicht, das nicht durch das Endoskop in die Vorrichtung gelangt, zu minimieren.

Es ist weiterhin zweckmäßig, daß zumindest eine der Linsen der Linsenanordnung eine Vergrößerungslinse ist und die Linsenanordnung eine Brennweite zwischen 5 und 100 Millimeter aufweist.

Weitere zweckmäßige Ausgestaltungen und Vorteile der Erfindung sind Gegenstand der nachfolgenden Beschreibung von Ausführungsbeispielen der Erfindung unter Bezug auf die Figuren der Zeichnungen. Es zeigen:
- Fig. 1: Eine geschnittene Ansicht eines Ausführungsbeispiels der erfindungsgemäßen Vorrichtung mit einem zu prüfenden Endoskop,
- Fig. 2: eine geschnittene Ansicht der Vorrichtung gemäß Fig. 1 mit einer Verschiebeeinheit und
- Fig. 3: eine Ansicht der Vorrichtung gemäß Fig. 1 von unten.

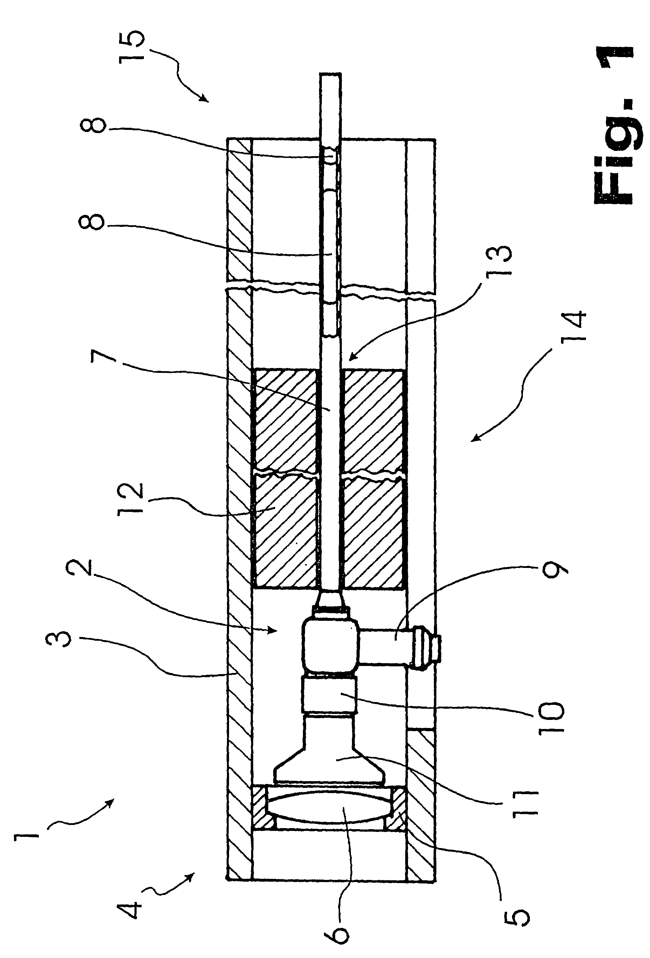

Fig. 1 zeigt eine geschnittene Ansicht eines Ausführungsbeispiels der erfindungsgemäßen Vorrichtung 1 mit einem zur Prüfung vorgesehen Endoskop 2. Die Vorrichtung 1 umfaßt eine als Führungsrohr 3 ausgestaltete Führungseinheit, die an ihrem proximalen Ende 4 über eine Linsenhalterung 5 verfügt. Die Linsenhalterung 5 ist zur Halterung einer als bikonvexe Vergrößerungslinse 6 ausgebildeten Linsenanordnung eingerichtet, wobei die Vergrößerungslinse 6 derart gehalten ist, daß ihre optische Achse mittig zum Führungsrohr 3 ausgerichtet ist. Der Brennwert der Vergrößerungslinse 6 beträgt in diesem Ausführungsbeispiel 20 Millimeter.

Das Endoskop 2 weist ein aus dem Führungsrohr 3 hervorragendes Hüllrohr 7 auf, in dem hintereinander durch nicht gezeigte Abstandshülsen voneinander beabstandete Stablinsen 8 angeordnet sind. An dem Hüllrohr 7 ist ein Kaltlichtkabelansatz 9 befestigt, der sich im wesentlichen quer zum Hüllrohr 7 erstreckt und an seiner vom Hüllrohr 7 abgewandten Seite über eine Okularhalterung 10 mit einem Endoskopokular 11 verbunden ist.

Zur Aufnahme und Fixierung des Endoskopes 2 verfügt die Vorrichtung 1 über ein als Gleitkörper 12 ausgebildetes Halteteil, das kreiszylindrisch ausgestaltet und dessen Außendurchmesser an den Innendurchmesser des Führungsrohres 3 angepaßt ist. Der Gleitkörper 12 weist eine sich mittig durch ihn hindurch erstreckende Durchgangsöffnung 13 auf, die zur Aufnahme des Hüllrohres 7 eingerichtet ist, um das Endoskop 2 in seiner Längsrichtung bezüglich der optischen Achse der Vergrößerungslinse 6 auszurichten.

Der Innendurchmesser des Führungsrohres 3 ist so bemessen, daß das Endoskop 2 mit seinem Endoskopokular 11 vollständig in das Führungsrohr 3 einführbar ist. Um Behinderungen seitlich abstehender Teile des Endoskopes 2 beim Einführen und Verschieben zu vermeiden, weist das Führungsrohr 3 eine in seine Rohrwandung eingeschnittene Ausnehmung 14 auf, die sich von seinem distalen Ende 15 in Längsrichtung des Führungsrohres 3 im wesentlichen bis zur Vergrößerungslinse 6 hin erstreckt. Die Ausnehmung 14 dient zur Aufnahme des sich quer zum Hüllrohr erstreckenden Kaltlichtkabelansatzes 9 und vermeidet auf diese Weise eine Behinderung der axialen Bewegung des Endoskopes 2 in dem Führungsrohr 3.

In einem abweichenden, nicht gezeigten Ausführungsbeispiel der erfindungsgemäßen Vorrichtung ist der Innendurchmesser des Führungsrohres 3 derart groß ausgestaltet, daß das Endoskop zusammen mit dem Kaltlichtkabelansatz in das Führungsrohr einführbar und darin verschiebbar ist, ohne dabei von der Rohrwandung behindert zu werden, so daß auf eine Ausnehmung in der Rohrwandung verzichtet werden kann.

Die Durchmesser des Gleitkörpers 12 beziehungsweise der Durchgangsöffnung 13 wurden aufeinander abgestimmt, so daß nach Einfügen sowohl des Gleitkörpers 12 in das Führungsrohr 3 als auch des Hüllrohres 7 in die Durchgangsöffnung 13 zwischen dem Hüllrohr 7 und der Durchgangsöffnung 13 ein größerer Reibungswiderstand besteht als zwischen dem Gleitkörper 12 und dem Führungsrohr 3. Eine axiale Bewegung des Endoskopes 2 bewirkt demzufolge eine axiale Verschiebung des Gleitkörpers 12 in dem Führungsrohr 3.

Durch die zum Führungsrohr 3 formangepaßte Ausgestaltung des Gleitkörpers 12 bleibt die Ausrichtung des Endoskopes 2 zur optischen Achse der Vergrößerungslinse 6 auch bei einer axialen Verschiebung des Gleitkörpers 12 in dem Führungsrohr 3 erhalten, so daß ein erneutes Ausrichten des Endoskopes 2 entfällt.

Zur Prüfung richtet ein Bediener das Endoskop 2 mit seinem Hüllrohr 7 auf eine hell beleuchtete Fläche hin aus und blickt in die Vergößerungslinse 6 hinein. In dem gezeigten Ausführungsbeispiel sind der Gleitkörper 12 und das Führungsrohr 3 aus einem lichtundurchlässigen Material hergestellt, so daß nach dem Einfügen des Gleitkörpers 12 und des Endoskopes 2 in das Führungsrohr 3 Licht im wesentlichen durch das Endoskop in die Vorrichtung 1 gelangt und störendes Fremdlicht vom Betrachter abgeschirmt ist.

Durch Verschieben des Endoskopes 2 von Hand an dem aus dem Führungsrohr 3 hervorragenden Abschnitt variiert der Betrachter den Abstand zwischen dem Endoskopokular 11 und der Vergrößerungslinse 6 solange, bis eine scharfe Abbildung einer Stablinse 8 erkennbar ist, um diese auf Trübungen, Linsenbrüche oder dergleichen hin zu untersuchen. Durch anschließende Abstandsänderungen können weitere Stablinsen 8 des Hüllrohres 7 vergrößert auf der Betrachternetzhaut abgebildet und untersucht werden. Während Verunreinigungen durch mehr oder weniger großflächige schwarze Punkte auszumachen sind, lassen sich Linsenbrüche in der Regel durch linienhafte Bruchstellen erkennen.

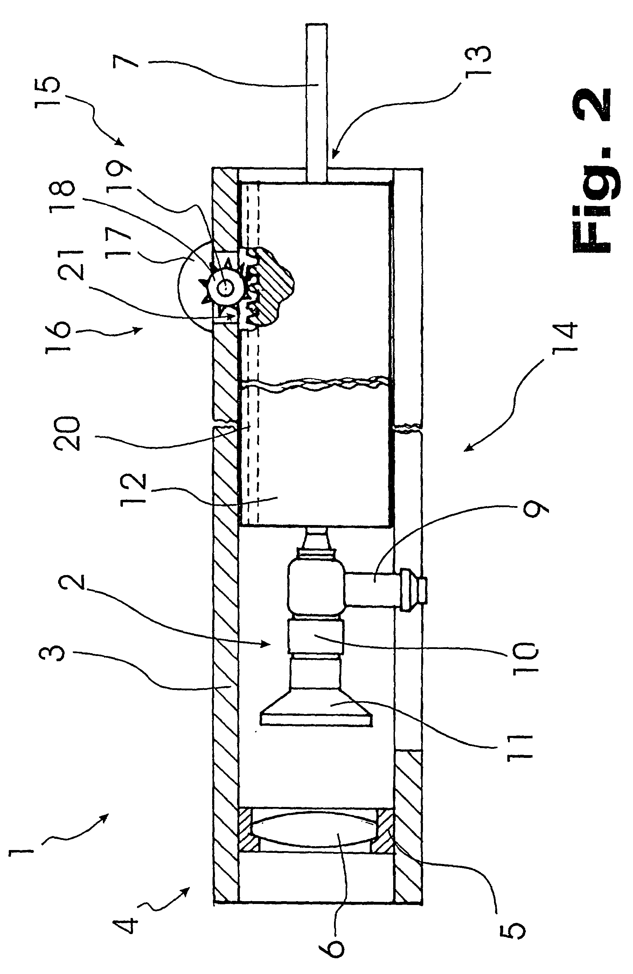

Fig. 2 zeigt einen Schnitt der Vorrichtung 1 gemäß Fig. 1 mit einem zu prüfenden Endoskop 2, wobei die Vorrichtung 1 zum Verfahren des Gleitkörpers 12 eine zusätzliche Verschiebeeinheit 16 aufweist, die ein Stellrad 17 und ein Zahnrad 18 umfaßt. Das Stellrad 17 ist außerhalb des Führungsrohres 3 angeordnet und mit dem in der Rohrwandung drehbar gelagerten Zahnrad 18 über eine Stellradwelle 19 drehfest verbunden. Der Gleitkörper 12 weist eine sich in seiner Längsrichtung erstreckende Nut 20 mit einem Zahnprofil 21 auf, in das das Zahnrad 20 der Verschiebeeinheit 16 eingreift.

Eine Drehung des Stellrades 17 von Hand variiert kontrollierbar den Abstand zwischen dem Endoskopokular 11 und der Vergrößerungslinse 6 und ermöglicht damit eine gegenüber dem in Fig. 1 gezeigten Ausführungsbeispiel genauere Einstellung der Abbildungsschärfe.

In einem abweichenden, nicht gezeigten Ausführungsbeispiel weist die Nut 20 eine glatte Oberfläche auf, wobei das antreibende Zahnrad als ein mit Gummi beschichtetes Verschieberad ausgebildet ist, das mit der glatten Bodenwandung der Nut reibschlüssig verbunden ist. Das Stellrad ist wiederum drehbar in der Wandung des Führungsrohres 3 gelagert. Ein Verschieben des Gleitkörpers 12 erfolgt nunmehr durch die direkte Drehung des Verschieberades von Hand, wobei aufgrund der glatten Oberfläche auf ein zusätzliches Stellrad verzichtet werden kann.

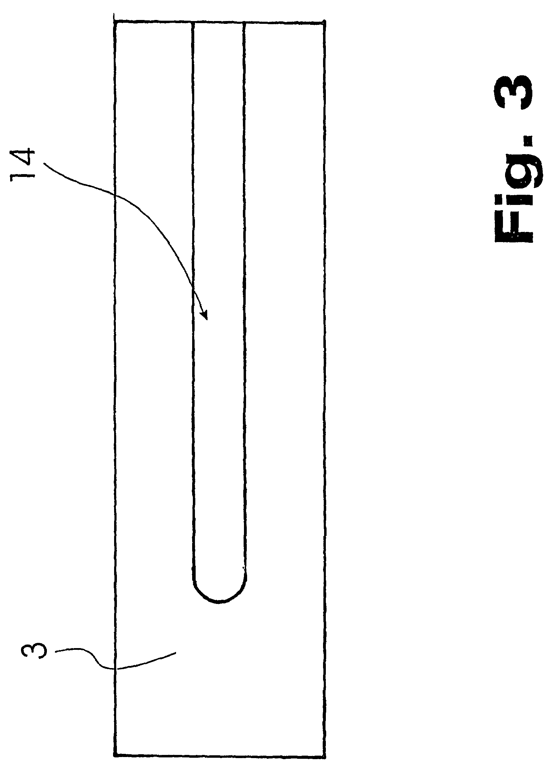

Fig. 3 zeigt die erfindungsgemäße Vorrichtung gemäß Fig. 1 von unten und verdeutlicht insbesondere die in die Rohrwandung des Führungsrohres 3 eingeschnittene Ausnehmung 14 zur Aufnahme sich vom Endoskop 2 in Querrichtung erstreckender Teile. Durch die Ausnehmung 14 ist eine Behinderung der axialen Verschiebung beispielsweise durch den Kaltlichtkabelansatz 9 vermeidbar.

In einem nicht gezeigten Ausführungsbeispiel weist die erfindungsgemäße Vorrichtung zwei in die Rohrwandung eingeschnittene Ausnehmungen zur Aufnahme von Endoskopteilen auf, die sich in quer zum Endoskop erstrecken und bezüglich der Längsachse des Endoskops einen Winkel von neunzig Grad aufspannen.

In einem nicht gezeigten weiteren Ausführungsbeispiel ist an dem proximalen Ende der erfindungsgemäßen Vorrichtung eine weitere Halterung zum Anbringen einer Dokumentationseinheit vorgesehen, die zum Aufnehmen und Speichern der bei dem Prüfverfahren gewonnenen Bilder eingerichtet ist. Zur Anpassung der erfindungsgemäßen Vorrichtung an die jeweils verwendete Dokumentationseinheit mit unterschiedlichen Eingangsoptiken ist die Vergrößerungslinse mittels einer geeigneten Linsenhalterung axial verfahrbar. Dazu weist die Linsenhalterung einen Einstellhebel auf, über den ein Bediener das Verfahren der Linsenanordnung einleitet. Die Ausnehmung 14 erstreckt sich in diesem Ausfühungsbeipiel durchgängig über die die gesamte Führungseinheit hinweg, um das Herausführen des Einstellhebels aus dem Inneren des Führungselementes nach außen zu ermöglichen.

## Patentansprüche

1. Vorrichtung zur Prüfung und Zustandsbeurteilung von Endoskopen (2) mit einer zur Aufnahme des zu prüfenden Endoskopes (2) vorgesehenen Führungseinheit (3), einer Linsenanordnung (6), die als Okular am proximalen Ende (4) der Führungseinheit (3) angeordnet ist, und einem in der Führungseinheit (3) in Richtung der optischen Achse der Linsenanordnung (6) verfahrbaren Halteteil (12) zur Aufnahme und Fixierung des Endoskopes (2).

2. Vorrichtung nach Anspruch 1, **dadurch gekennzeichnet, daß** die Führungseinheit als Führungsrohr (3) ausgebildet ist.

3. Vorrichtung nach Anspruch 2, **dadurch gekennzeichnet, daß** das Halteteil ein kreiszylinderförmiger Gleitkörper (12) ist, dessen Außendurchmesser an den Innendurchmesser des Führungsrohres (3) angepaßt ist, und der eine mittige Durchgangsöffnung (13) zum Einführen und Fixieren eines Hüllrohres (7) des zu prüfenden Endoskopes (2) aufweist, wobei der Innendurchmesser der Durchgangsöffnung (13) an den Außendurchmesser des Hüllrohres (7) angepaßt ist.

4. Vorrichtung nach Anspruch 3, **dadurch gekennzeichnet, daß** der Gleitkörper (12) eine in seine Längsrichtung verlaufende Nut (20) aufweist, die mit ihrer Bodenwandung an einem in der Wandung des Führungsrohres (3) drehbar gelagerten Verschieberad (18) anliegt, so daß der Gleitkörper (12) reibschlüssig mit dem Verschieberad verbunden ist, wobei das Verschieberad (18) die Wandung des Führungsrohres (3) umfänglich überragt und durch seine von Hand antreibbare Drehung das Verfahren des Gleitkörpers (12) in dem Führungsrohr (3) bewirkt.

5. Vorrichtung nach Anspruch 4, **dadurch gekennzeichnet, daß** die Nut (20) einen Zahnprofilbereich (21) aufweist, in den das als Zahnrad (18) ausgebildete Verschieberad eingreift, wobei das Zahnrad (18) über eine Stellradwelle (19) drehfest mit einem außerhalb des Führungsrohres (3) gelagerten Stellrad (17) verbunden ist.

6. Vorrichtung nach Anspruch 5, **dadurch gekennzeichnet, daß** die Stellradwelle (19) mit einer auf dem Führungsrohr (3) befestigten Antriebseinheit gekoppelt ist, die einen von einem Energiespeicher gespeisten Elektromotor, Getriebemittel und einen aus der Antriebseinheit hervorragenden, federnden Druckkontakt umfaßt, der während eines von außen aufgebrachten Druckes das Einleiten einer von dem Elektromotor erzeugten Drehbewegung auf die Stellradwelle (19) und damit eine Verschiebung des Gleitkörpers (12) in dem Führungsrohr (3) bewirkt.

7. Vorrichtung nach einem der vorhergehenden Ansprüche, **dadurch gekennzeichnet, daß** das Führungselement (3) zumindest eine in seine Rohrwandung eingeschnittene Ausnehmung (14) aufweist, die sich von seinem distalen Ende (15) in Längsrichtung erstreckt.

8. Vorrichtung nach Anspruch 7, **dadurch gekennzeichnet, daß** sich zumindest eine Ausnehmung (14) durchgängig, über das gesamte Führungselement (3) hinweg erstreckt.

9. Vorrichtung nach Anspruch 8, **dadurch gekennzeichnet, daß** sich zumindest eine Ausnehmung (14) bis zur Linsenanordnung (6) hin erstreckt.

10. Vorrichtung nach einem der vorhergehenden Ansprüche, **dadurch gekennzeichnet, daß** die Führungseinheit (3) und das Halteteil (12) aus einem lichtabschirmenden Material hergestellt sind.

11. Vorrichtung nach einem der vorhergehenden Ansprüche, **dadurch gekennzeichnet, daß** zumindest eine der Linsen der Linsenanordnung eine Vergrößerungslinse (6) ist.

12. Vorrichtung nach einem der vorhergehenden Ansprüche, **dadurch gekennzeichnet, daß** die Linsenanordnung (6) eine Brennweite zwischen 5 und 100 Millimetern aufweist.

## Claims

1. Device for testing and evaluating the condition of endoscopes (2), with a guide unit (3, provided to receive the endoscope (2) to be tested, a lens arrangement (6) arranged as an eyepiece at the proximal end (4) of guide unit (3), and a holding part (12) to receive and fix the endoscope (2), said holding part (12) being displaceable in guide unit (3) in the direction of the optical axis of lens arrangement (6).

2. Device according to Claim 1, **characterised in that** the guide unit is formed as a guide tube (3).

3. Device according to Claim 2, **characterised in that** the holding part is a regular cylindrical sliding body (12) the external diameter of which is adapted to the internal diameter of guide tube (3), and which has a central through hole (13) for inserting and fixing a sheathing tube (7) of the endoscope (2) to be tested, the internal diameter of through hole (13) being adapted to the external diameter of the sheathing tube (7).

4. Device according to Claim 3, **characterised in that** sliding body (12) has a groove (20) running in its longitudinal direction, the bottom face of which rests against a displacing wheel (18) rotatably mounted in the wall of guide tube (3), so that sliding body (12) is frictionally engaged with the displacing wheel, displacing wheel (18) projecting circumferentially beyond the wall of guide tube (3) and causing sliding body (12) to be displaced in guide tube (3) by its manually driveable rotation.

5. Device according to Claim 4, **characterised in that** groove (20) has an area with a toothed profile (21) into which the displacing wheel, formed as a gear wheel (18), engages, gear wheel (18) being non-rotatably connected to an adjusting wheel (17) located outside guide tube (3) by means of an adjusting wheel shaft (19).

6. Device according to Claim 5, **characterised in that** adjusting wheel shaft (19) is coupled to a drive unit fixed to guide tube (3), which drive unit includes an electric motor supplied by an energy accumulator, transmission means and a resilient pressure contact projecting outside the drive unit, which pressure contact, while pressure is applied to it from outside, causes a rotary motion generated by the electric motor to be applied to adjusting wheel shaft (19) and therefore causes sliding body (12) to be displaced in guide tube (3).

7. Device according to one of the preceding claims, **characterised in that** guide element (3) has at least one opening (14) cut in its tubular wall and extending longitudinally from its distal end (15).

8. Device according to Claim 7, **characterised in that** at least one opening (14) extends along the whole of guide element (3).

9. Device according to Claim 8, **characterised in that** at least one opening (14) extends as far as lens arrangement (6).

10. Device according to one of the preceding claims, **characterised in that** guide unit (3) and holding part (12) are made of a light-screening material.

11. Device according to one of the preceding claims, **characterised in that** at least one of the lenses of the lens arrangement is a magnifying lens (6).

12. Device according to one of the preceding claims, **characterised in that** lens arrangement (6) has a focal length between 5 and 100 millimeters.

## Revendications

1. Dispositif pour la vérification et l'évaluation de l'état d'endoscopes (2) comportant une unité de guidage (3), prévue pour recevoir l'endoscope (2) à examiner, un ensemble de lentilles (6) qui est disposé en tant qu'oculaire à l'extrémité proximale (4) de l'unité de guidage (3), ainsi qu'un élément de maintien (12) déplaçable dans l'unité de guidage (3), en direction de l'axe optique de l'ensemble de lentilles (6), pour recevoir et fixer l'endoscope (2).

2. Dispositif selon la revendication 1, **caractérisé en ce que** l'unité de guidage est réalisée en tant que tube de guidage (3).

3. Dispositif selon la revendication 2, **caractérisé en ce que** l'élément de maintien est un corps glissant (12) en forme de cylindre circulaire dont le diamètre extérieur est adapté au diamètre intérieur du tube de guidage (3), et qui présente un orifice de passage central (13) pour l'introduction et la fixation d'un tube d'enveloppe (7) de l'endoscope (2) à examiner, le diamètre intérieur de l'orifice de passage (13) étant adapté au diamètre extérieur du tube d'enveloppe (7).

4. Dispositif selon la revendication 3, **caractérisé en ce que** le corps glissant (12) présente une rainure (20) qui s'étend dans sa direction longitudinale et qui s'applique, par sa paroi de fond, contre une roue de coulissement (18) montée tournante dans la paroi du tube de guidage, ce qui fait que le corps glissant (12) est relié à friction à la roue de coulissement, la roue de coulissement (18) dépassant périphériquement de la paroi du tube de guidage (3) et provoquant, par sa rotation, à commander manuellement, le déplacement du corps glissant (12) dans le tube de guidage (3).

5. Dispositif selon la revendication 4, **caractérisé en ce que** la rainure (20) présente une zone de profil denté (21) dans laquelle s'engage la roue de coulissement, réalisée en tant que roue dentée (18), la roue dentée (18) étant reliée solidairement en rotation, par un arbre de roue de réglage (19), à une roue de réglage (17) montée à l'extérieur du tube de guidage (3).

6. Dispositif selon la revendication 5, **caractérisé en ce que** l'arbre de roue de réglage (19) est accouplé à une unité d'entraînement, fixée sur le tube de guidage (3) et qui comprend un moteur électrique alimenté par un accumulateur d'énergie, des moyens de transmission et un contact de compression élastique, dépassant de l'unité d'entraînement, qui, pendant une pression appliquée de l'extérieur, provoque l'amorce d'un mouvement de rotation, produit par le moteur électrique, sur l'arbre de roue de réglage (19) et ainsi un coulissement du corps glissant (12) dans le tube de guidage (3).

7. Dispositif selon l'une des revendications précédentes, **caractérisé en ce que** l'élément de guidage (3) s'étend dans la direction longitudinale.

8. Dispositif selon la revendication 7, **caractérisé en ce qu'**au moins un évidement (14) s'étend de part en part sur tout l'élément de guidage (3).

9. Dispositif selon la revendication 8, **caractérisé en ce qu'**au moins un évidement (14) s'étend jusqu'à l'ensemble de lentilles (6).

10. Dispositif selon l'une des revendications précédentes, **caractérisé en ce que** l'unité de guidage (3) et l'élément de maintien (2) sont réalisés dans une matière faisant écran à la lumière.

11. Dispositif selon l'une des revendications précédentes, **caractérisé en ce qu'**au moins l'une des lentilles de l'ensemble de lentilles est une lentille de grossissement (6).

12. Dispositif selon l'une des revendications précédentes, **caractérisé en ce que** l'ensemble de lentilles (6) présente une distance focale comprise entre 5 et 100 millimètres.
